(19) Europäisches Patentamt
European Patent Office
Office européen des brevets

(11) **EP 3 632 330 B1**

(12) **EUROPEAN PATENT SPECIFICATION**

(45) Date of publication and mention of the grant of the patent:
**24.08.2022 Bulletin 2022/34**

(21) Application number: **18306309.8**

(22) Date of filing: **04.10.2018**

(51) International Patent Classification (IPC):
*A61B 8/00* (2006.01)     *A61B 8/08* (2006.01)
*G01H 5/00* (2006.01)

(52) Cooperative Patent Classification (CPC):
**G01H 5/00; A61B 8/085; A61B 8/0858;
A61B 8/5207;** A61B 8/485

(54) **METHOD FOR DETERMINING A SPEED OF SOUND IN A MEDIUM, AN ULTRASOUND IMAGING SYSTEM IMPLEMENTING SAID METHOD**

VERFAHREN ZUR BESTIMMUNG EINER SCHALLGESCHWINDIGKEIT IN EINEM MEDIUM, ULTRASCHALLBILDGEBUNGSSYSTEM ZUR DURCHFÜHRUNG DES BESAGTEN VERFAHRENS

PROCÉDÉ DE DÉTERMINATION DE LA VITESSE DU SON DANS UN MILIEU, SYSTÈME D'IMAGERIE PAR ULTRASONS METTANT EN  UVRE LEDIT PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.04.2020 Bulletin 2020/15**

(73) Proprietor: **SuperSonic Imagine
13857 Aix-en-Provence Cedex 3 (FR)**

(72) Inventor: **COUADE, Mathieu
13100 Aix-en-Provence (FR)**

(74) Representative: **Plasseraud IP
66, rue de la Chaussée d'Antin
75440 Paris Cedex 09 (FR)**

(56) References cited:
**US-A1- 2011 082 372     US-A1- 2013 178 740
US-A1- 2018 125 451**

**Description**

**FIELD OF THE INVENTION**

More particularly, for the invention, the medium comprises an outer surface, an intermediate region situated under said outer surface and a target region situated under the intermediate region.

The method aims to determine a speed of sound inside the target region by using an ultrasound system having a probe that is put into contact with outer surface of the medium.

**BACKGROUND OF THE INVENTION**

The method of the invention may be applied to determining the speed of sound inside a mammal body liver so as, in one example, to identify and quantify liver hepatic steatosis.

The hepatic steatosis is an accumulation of fat inside the liver. It is usually determined by magnetic resonance imaging (MRI). The magnetic resonance imaging allows measuring a quantity of fat medium, but using MRI is costly and implies use of rare resources. Another diagnostic method consists in an invasive liver biopsy and then histology.

Another way to determine the accumulation of fat inside the liver is by evaluating a speed of sound in the liver. The speed of sound indeed inside a tissue varies with the amount of fat present in the tissue. The speed of sound in fat is lower than in a healthy liver. As a result, a steatosis liver has a speed of sound slightly lower than that of a healthy liver. For example, the speed of sound in a steatosis liver may typically be decreased from 1580 m/s to 1460 m/s compared to that of a healthy liver. In proportion, such decrease represents only 5-10%. It may as a consequence be difficult to identify a speed characterizing a fatty liver of one having marginal errors of measurement.

Therefore, to detect an actual hepatic steatosis, the speed of sound should be measured as accurately as possible.

Because the liver is located inside the body in a region underneath a surface intermediate region that comprises the skin, some fat, and eventually intercostal muscles, measurements may be impaired by the presence of the intermediate surface region.

The document "Robust sound speed estimation for ultrasound-based hepatic steatosis assessment", Marion Imbault et Al, Physics in Medecine & Biology, 2017, discloses a way of measuring a global speed of sound in the medium by applying correction of aberrations occurring during the propagation of ultrasound waves in the medium. The method then uses a virtual point source generation inside the medium and an iterative focusing algorithm to insure that the virtual point source is well focused. Then, the global speed of sound is estimated. After this first step, the speed of sound in the liver is then calculated by assuming the thickness and speed of sound in the fat and in the muscle are well known. These can be determined using reference (average) values taken from the literature (for example of the speed of sound) and/or manual estimations could be used (for example for the thickness). The manual estimation has the drawback to be operator dependent.

However, such a technique requires several iterations to process the aberration correction, and the thickness and speed of sound in the fat and muscle must be known. Finally, such method does not take correctly into account the inhomogeneity of the medium in the upper region, and an additional computation to correct the measured value and to determine the speed of sound in the liver is mostly arbitrary.

The document US 2013/0178740 A1, according to its abstract, discloses a method of providing an ultrasound image including: obtaining an ultrasound image of an object; detecting a plurality of layers having different sound speeds based on the ultrasound image; and displaying information about boundaries of the plurality of layers.

The document US 2011/082372 A1, according to its abstract, discloses an ultrasonic diagnostic apparatus provided with a boundary extraction unit that extracts boundaries between media appearing in an ultrasonic image based on the intensity of each of received signals. A boundary extraction unit performs boundary extraction processing on each of individual time series ultrasonic images sequentially generated while operating an ultrasonic probe to move, sets a target region (margin region) matched to a movement operation of the ultrasonic probe in an ultrasonic image being processed from a boundary extracted in an ultrasonic image preceding the ultrasonic image being processed, and performs processing of extracting a boundary in the ultrasonic image being processed by using received signals corresponding to the reflection waves reflected at an inner side of the target region thus set.

The document US 2018/012545 A1, according to its abstract, discloses a device for sound speed imaging where different receive apertures are used instead of multiple transmissions from different angles. Acoustic echoes from a same transmission are received beamformed with different apertures of the transducer array. The axial shift between the beamformed signals from the different apertures is used to solve for the speed of sound at one or more locations. The resulting measure of the speed of sound is displayed as the speed of sound in the tissue and may be diagnostically or prognostically useful.

Consequently, there is a need for a more accurate way to determine a speed of sound in an inner region of a mammal part.

## OBJECTS AND SUMMARY OF THE INVENTION

One object of the present invention is to provide a **method of determining the speed of sound inside a target region** of a medium (that is a region underneath a intermediate region) without having predetermined information of the intermediate region.

The method is implemented by using an ultrasound imaging system comprising at least a probe adapted to sense backscattered waves and to provide sensed signals corresponding to said backscattered waves to a processing unit (16) of the ultrasound system.

The method comprises the following steps:

- determining on a morphological image a position of at least an interface in the medium, the interface dividing the medium into an intermediate region of the medium and the target region in a depth direction,
- determining a first speed of sound of the intermediate region based on at least some of the sensed signals, and
- determining the target speed of sound inside the target region based on at least some of the sensed signals and taking into account the position of the interface, , and the first speed of sound.

Thanks to these features, the method builds a inhomogeneous model of the medium having an interface, a first speed of sound and a target speed of sound. Such method permits to obtain an accurate estimation of the target speed of sound.

This accurate determination of target speed of sound can then be used to detect small variations of speed of sound in the target region compared to reference healthy speed of sound value for identical medium. This accurate method permits to use ultrasound technique for relevant detection of disease, such as hepatic steatosis.

In various embodiments of the method, one and/or other of the following features may optionally be incorporated.

In one aspect of the method, the probe is adapted to be functionally put in contact with an outer surface of the medium, the probe being adapted to transmit excitation waves into the medium in a depth direction toward the target region, said excitation waves being backscattered in the medium toward the probe.

In one aspect of the method:

- the first speed of sound is determined by taking into account a first reference speed of sound, and one of a plurality of first supposed speeds of sound, and
- the target speed of sound is determined by taking into account the position of the interface, a target reference speed of sound applied to the target region, the first speed of sound applied to the intermediate region, and one of a plurality of supposed target speeds of sound for the target region.

In one aspect of the method, the method further comprises a step of calculating a plurality of first image data associated with a first representative area of the intermediate region before determining the first speed of sound of the intermediate region, the first speed of sound being based on the plurality of first image data, each of the first image data being determined based on a beamforming algorithm applied to at least the sensed signals corresponding to the first representative area and which takes as a parameter a first reference speed of sound, and one of a plurality of first supposed speeds of sound.

In one aspect of the method, the method further comprises a step of calculating a plurality of target image data associated with a target representative area of the target region before determining the target speed of sound) inside the target region, the target speed of sound being based on the plurality of target image data in the representative area of the target region, each of the target image data being determined based on a beamforming algorithm applied to at least the sensed signals corresponding to the representative area and which takes as parameters the position of the interface, the target reference speed of sound applied to the target region, the first speed of sound applied to the intermediate region, and one of the plurality of supposed target speeds of sound for the target region.

In one aspect of the method, at least one of:

- the plurality of first supposed speeds of sound and the plurality of supposed target speeds of sound have a same value, and
- the first supposed speeds of sound are known speeds of sound for the intermediate region, and the supposed target speeds of sound are known speeds of sound for the target region.

In one aspect of the method, the determination of the position of the interface is based on at least some of the sensed signals.

In one aspect of the method, the determination of the position of the interface is based on an automatic image processing of the morphological image.

In one aspect of the method, the position of the interface is determined based on variations of amplitudes of image data of the medium along the depth direction between the intermediate region and the target region, the image data being determined based on the sensed signals and a beamforming algorithm which takes as a parameter the reference speed of sound.

In one aspect of the method, the first speed of sound and/or the target speed of sound are each calculated using a respective first and/or target focusing criterion, a plurality of respective first and/or target focusing values being obtained by applying the respective first and/or target focusing criterion to, respectively, the plurality of first image data of the first representative area and/or the plurality of target image data of the target representative area, the first speed of sound being a selected one of the plurality of respective first focusing values and/or the target speed of sound being a selected one of the plurality of respective target focusing values.

In one aspect of the method, the first speed of sound is the maximum of the plurality of respective first focusing values and/or the target speed of sound is the maximum of the plurality of respective target focusing values.

In one aspect of the method, the focusing criterion is a coherence criterion.

In one aspect of the method, the method further comprises the steps of:

- determining a position of a sub-interface in the intermediate region, the sub-interface dividing in the depth direction the intermediate region into a second region of the intermediate region proximal to the outer surface and a first region of the intermediate region proximal to the interface,
- determining a second speed of sound of the second region based on at least some of the sensed signals and taking as a parameter a second reference speed of sound, and one of a plurality of second supposed speeds of sound, wherein

the determination of the first speed of sound is based on at least some of the sensed signals and takes into account the position of the sub-interface, the first reference speed of sound applied to the first region, the second reference speed of sound applied to the second region, and one of the plurality of first supposed speeds of sound for the first region.

In one aspect of the method, the determination of the position of the sub-interface being based on at least some of the sensed signals.

In one aspect of the method, the second region of the intermediate region contains the second representative area, and the first region of the intermediate region contains the first representative area.

In one aspect of the method, the method further comprises a step of calculating a plurality of second image data associated with a second representative area of the second region before determining the second speed of sound of the second region based on the plurality of second image data, each of the second image data being determined based on a beamforming algorithm applied to at least the sensed signals corresponding to the second representative area and which takes as a parameter a second reference speed of sound, and one of a plurality of second supposed speeds of sound.

In one aspect of the method, the medium is a mammal body and the outer surface is the skin of the mammal, and the target region is the liver of the mammal, the intermediate region is a region of the medium comprised between the liver and the skin in the depth direction.

An other object of the present invention is to provide **an ultrasound imaging system for determining a target speed of sound** inside a target region of a medium, said ultrasound imaging system comprising:

- a probe adapted to sense backscattered waves and to provide corresponding sensed signals to the ultrasound system, and
- a processing unit configured to:

  - determine a position of at least an interface in the medium on a morphological image, the interface dividing the medium into an intermediate region of the medium and the target region in the depth direction,

  - determine a first speed of sound of the intermediate region based on at least some of the sensed signals, and
  - determine the target speed of sound inside the target region based on at least some of the sensed signals and taking into account the position of the interface, and the first speed of sound.

In various embodiments of the system, one and/or other of the following features may optionally be incorporated.

In one aspect of the system, the probe is adapted to be functionally put in contact with an outer surface of the medium, the probe being adapted to transmit excitation waves into the medium in a depth direction toward the target region, said excitation waves being backscattered in the medium toward the probe.

In one aspect of the system:

- the first speed of sound is determined by taking into account a first reference speed of sound, and one of a plurality of first supposed speeds of sound, and
- the target speed of sound is determined by taking into account the position of the interface, a target reference speed of sound applied to the target region, the first speed of sound applied to the intermediate region, and one of a plurality of supposed target speeds of sound for the target region.

In one aspect of the system, the system further calculates a plurality of first image data associated with a first representative area of the intermediate region before determining the first speed of sound of the intermediate region, the first speed of sound being based on the plurality of first image data, each of the first image data being determined based on a beamforming algorithm applied to at least the sensed signals corresponding to the first representative area and which takes as a parameter a first reference speed of sound, and one of a plurality of first supposed speeds of sound.

In one aspect of the system, the system further calculates a plurality of target image data associated with a target representative area of the target region before determining the target speed of sound) inside the target region, the target speed of sound being based on the plurality of target image data in the representative area of the target region, each of the target image data being determined based on a beamforming algorithm applied to at least the sensed signals corresponding to the representative area and which takes as parameters the position of the interface, the target reference speed of sound applied to the target region, the first speed of sound applied to the intermediate region, and one of the plurality of supposed target speeds of sound for the target region.

In one aspect of the system, at least one of:

- the plurality of first supposed speeds of sound and the plurality of supposed target speeds of sound have a same value, and
- the first supposed speeds of sound are known speeds of sound for the intermediate region, and the supposed target speeds of sound are known speeds of sound for the target region.

In one aspect of the system, the determination of the position of the interface is based on at least some of the sensed signals.

In one aspect of the system, the determination of the position of the interface is based on an automatic image processing of the morphological image.

In one aspect of the system, the position of the interface is determined based on variations of amplitudes of image data of the medium along the depth direction between the intermediate region and the target region, the image data being determined based on the sensed signals and a beamforming algorithm which takes as a parameter the reference speed of sound.

In one aspect of the system, the first speed of sound and/or the target speed of sound are each calculated using a respective first and/or target focusing criterion, a plurality of respective first and/or target focusing values being obtained by applying the respective first and/or target focusing criterion to, respectively, the plurality of first image data of the first representative area and/or the plurality of target image data of the target representative area, the first speed of sound being a selected one of the plurality of respective first focusing values and/or the target speed of sound being a selected one of the plurality of respective target focusing values.

In one aspect of the system, the first speed of sound is the maximum of the plurality of respective first focusing values and/or the target speed of sound is the maximum of the plurality of respective target focusing values.

In one aspect of the system, the focusing criterion is a coherence criterion.

In one aspect of the system, the system further:

- determines a position of a sub-interface in the intermediate region, the sub-interface dividing in the depth direction the intermediate region into a second region of the intermediate region proximal to the outer surface and a first region of the intermediate region proximal to the interface,
- determines a second speed of sound of the second region based on at least some of the sensed signals and taking as a parameter a second reference speed of sound, and one of a plurality of second supposed speeds of sound, wherein

the determination of the first speed of sound is based on at least some of the sensed signals and takes into account the position of the sub-interface, the first reference speed of sound applied to the first region, the second reference speed of sound applied to the second region, and one of the plurality of first supposed speeds of sound for the first region.

In one aspect of the system, the determination of the position of the sub-interface being based on at least some of the sensed signals.

In one aspect of the system, the second region of the intermediate region contains the second representative area, and the first region of the intermediate region contains the first representative area.

In one aspect of the system, the system further calculates a plurality of second image data associated with a second representative area of the second region before determining the second speed of sound of the second region based on the plurality of second image data, each of the second image data being determined based on a beamforming algorithm applied to at least the sensed signals corresponding to the second representative area and which takes as a parameter a second reference speed of sound, and one of a plurality of second supposed speeds of sound.

In one aspect of the system, the medium is a mammal body and the outer surface is the skin of the mammal, and the target region is the liver of the mammal, the intermediate region is a region of the medium comprised between the liver and the skin in the depth direction.

## BRIEF DESCRIPTION OF THE DRAWINGS

Other features and advantages of the invention will be apparent from the following detailed description of its embodiments given by way of non-limiting example, with reference to the accompanying drawings. In the drawings:

- Figure 1 is a schematic of a system for determining a speed of sound in a target region of a medium showing a schematic of the medium segmented into two regions;
- Figure 2 is an example of a visual image 25 of the medium obtained using the system of the Figure 1 revealing an intermediate region and the target region;
- Figure 3 is a flowchart of a method for determining the speed of sound in the target region of the medium;
- Figure 4 is an example of chart of coherence value versus speed of sound in the intermediate region of the medium;
- Figure 5 is an example of chart of coherence value versus speed of sound in the target region of the medium;
- Figure 6 is a schematic of the medium of Figure 1 showed segmented into three regions; and
- Figure 7 is an example of spatial autocorrelation calculated for estimating a resolution of an area in the image data.

## DETAILED DESCRIPTION

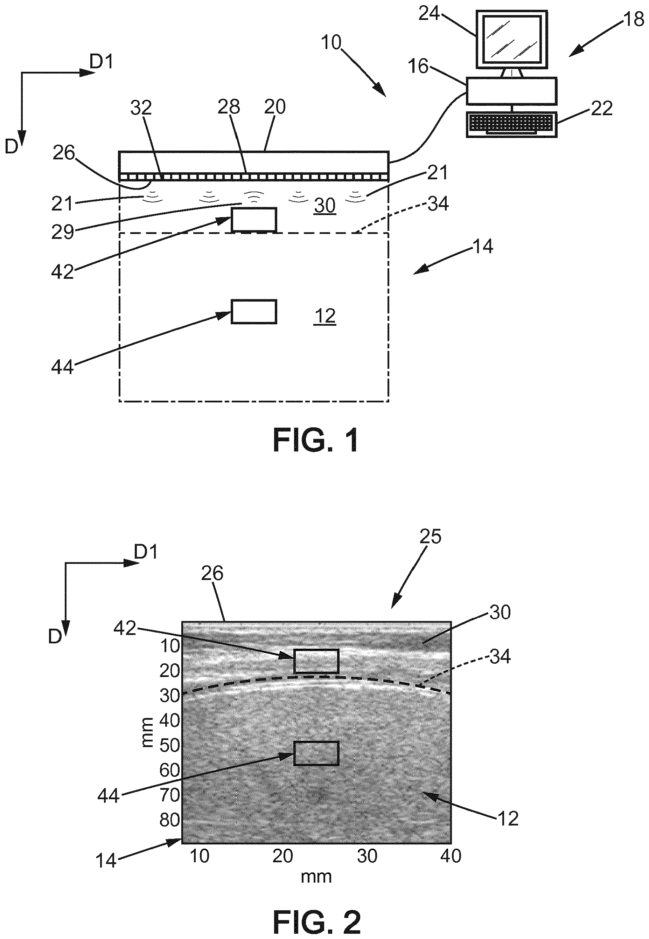

**Figure 1** is a schematic of an ultrasound imaging **system 10** for determining a speed of sound c, herein referred as **target speed of sound cT,** in a region of interest, herein referred as **target region 12,** of a **medium 14.** The schematic of the Figure 1 is not to scale and is merely provided to present the system 10 and to illustrate its functioning.

The medium 14 may be any part of a mammal body, such as a portion of a mouse or a human. In one embodiment, the medium 14 is an abdominal portion of a human containing the liver. In another embodiment, the medium 14 is a portion of breast of a human. In another embodiment, the medium 14 is a portion of a human brain.

The target region 12 is any inner region of the medium 14, which is a region located underneath a surface of the medium 14, i.e. distal from a surface of the medium 14. In one embodiment, the target region 12 is a portion of the liver contained in the abdominal portion.

The system 10 may be used to determine various characteristics of the medium 14, and in particular to determine the speed of sound in the target region 12.

The system 10 includes a **processing unit 16,** which in this example is shown embedded in a **computer system 18,** and a **probe 20** for transmitting and receiving **excitation waves 21** to and from the medium 14 (only a few schematic excitation waves 21 being represented in Figure 1). The processing unit 16 is functionally associated to the probe 20 so as to control the probe 20, but also to receive information collected by the probe 20.

The computer system 18 may include an interface to input instructions to the processing unit 16, such as a **keyboard 22.** The instructions are for examples a shape and intensity of the excitation waves 21. It is contemplated that the keyboard 22 may be replaced by a touch screen keyboard, and may be wirelessly associated to the processing unit 16. The computer system 18 may also include a **display 24** to visualize an **image 25** (referred herein as "visual image 25") of the medium 14 corresponding to the data collected by the probe 20. An example of such visual image 25 is shown in Figure 2. The display 24 may be wirelessly connected to the computer system 18. It is contemplated that the keyboard 22, the display 24 and/or the probe 20 could physically be remote from each other and from the processing unit 16. They could be for example in different rooms or locations, thus allowing a person remote of the probe 20 to analyze in real time or not the data collected by the probe 20.

The probe 20 is an element of the system 10 capable of transmitting and receiving excitation waves, in the form of ultrasound waves, to and from the medium 14 so that the processing unit 16 can at least process them into the visual image 25 representative of the medium 14. Such type of imaging is commonly known as echography. As will be described below, the processing unit 16 may further exploit the data corresponding to the received waves from the medium 14 to determine the speed of sound c in the target region 12.

In operation, as shown in figure 1, the probe 20 is functionally put in contact (i.e. directly or indirectly via an ultrasound gel) with an **outer surface 26** of the medium 14. If the medium 14 is an abdominal portion of a human body, the outer surface 26 is, for example, the skin.

The probe 20 includes a plurality of **transducers 28** adjacent to each other, which send each the excitation waves 21 (acoustic or ultrasonic waves) in a **depth direction D** into the medium 14 toward the target region 12. In general, the depth direction D is substantially perpendicular to a **surface of emission 32** of the probe 20. The surface of emission 32 extends in a **surface direction D1** substantially corresponding to the spatial distribution of the transducers 28. The transducers 28 may be aligned to form a line of transducers in the surface direction D1 or may form a planar 2D surface of transducers. The target region 12 may be deep inside the medium 14 so that the acoustic waves 21 first travel through the outer surface 26 and an **intermediate region 30** of the medium 14 before reaching the target region 12. For instance, if the medium 14 is an abdominal portion of a human body and the target region 12 is the liver, the intermediate region 30 could be made of muscle, connective tissues, and/or some fat. The intermediate region 30 influences the propagation of waves in the medium 14 and consequently any calculation of the speed of sound based on sensed signals from these waves.

The transducers 28 are adapted to send any type of waves into the medium 14, i.e. focused waves or unfocussed waves such as convergent, divergent or planar waves or quasi planar waves. In one embodiment, the transducers 28 are emitting series of planar waves having a penetration angle $\alpha$ with respect to the surface direction D1. To generate planar waves at a penetration angle $\alpha$, the transducers 28 are actuated with a time lag relative to each other.

The transducers 28 are also adapted to receive waves emitted by the medium 14. These are called **backscattered waves 29.** When the excitation waves 21 are transmitted into the medium 14, a portion of them is emitted back from the medium 14 toward the probe 20 when they encounter reflective elements in the medium. The reflective elements may be particles being heterogeneities of the medium. For example, these particles may be collagen particles that are included in any mammal tissue. These reflective elements generate in the visual image 25 points that are designated as "speckle". An intensity of these backscattered waves 29 can thus be used as a way to distinguish various tissues within the medium 14 to form the visual image 25 of the medium 14.

Referring more particularly now to **figure 2,** an example of visual image 25 determined from the data collected by the probe 20 (i.e. backscattered waves 29) is provided. The visual image 25 displays the medium 14 with one or more inhomogeneity. The medium 14 may be segmented as follow: a first quasi-homogenous region of the medium 14 corresponding to the target region 12 (the liver in this example); and a second non-homogeneous portion yet distinct from the target region 12 corresponding to the intermediate region 30. An **interface 34** may be identified between the target region 12 and the intermediate region 30. The interface 34 corresponds to the transition between tissues of different characteristics, such as composition or density. The position of the interface 34 will be taken into account in the calculation of the speed of sound c in the target region 12 (referred herein after as "target speed of sound cT"), as will be discussed below. Because the target region 12 (e.g. liver) is reached by the excitation waves 21 only after travelling through the intermediate region 30 (e.g. connective and muscle tissues), the calculation of the speed of sound in the target region 12 may be altered by the presence of the intermediate region 30.

The interface 34 divides the medium 14 into the target region 12 and the intermediate region 30. The target region 12 and the intermediate region 30 may be of different sizes. For example, the intermediate region 30 may be shallow compared to the target region 30.

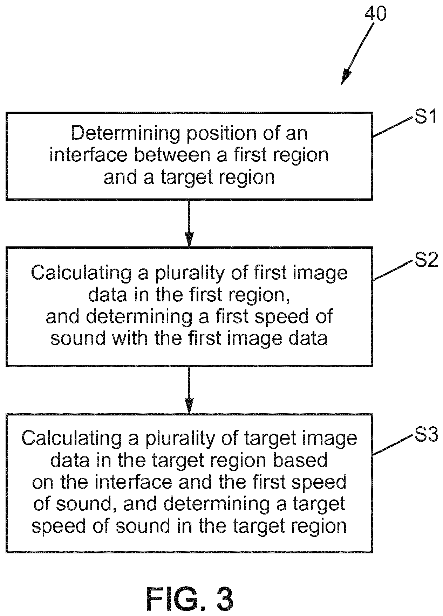

Referring now to **figure 3,** a **method 40,** according to a first embodiment, for determining the target speed of sound cT in the target region 12 will be described. The method 40 uses the ultrasound imaging system 10. The probe 20 is put in contact with the outer surface 26 of the medium 14 (e.g. skin) so as to transmit excitation waves 21 into the medium 14 in the depth direction D toward the target region 12 (e.g. liver). The excitation waves 21 are backscattered in the medium 14 toward the probe 20. The probe 20 senses the backscattered waves 29 and provides sensed signals corresponding to the backscattered waves 29 to the processing unit 16 of the ultrasound system 10. The sensed signals are time related signals commonly referred as "raw signals" or "RF signals". These signals are not beamformed signals. They are processed by the processing unit 16 to obtain information regarding the medium 14.

The image data is a collection of values obtained from the sensed signals which provide for each spatial location of the zone of the medium 14 covered by the transducers 26 an indication of the resistance to backscatter the excitations waves 21. In one example, the image data are used to form the visual image 25 of the medium 14. The image data is an image having preferably more than one pixel value, and for example more than ten pixel values. If the image data corresponds to a physical rectangular region in the medium, the image data can be a matrix of pixel values. This matrix can have a size of 3x3 pixels or more.

The processing of the sensed signals into image data (i.e. an ensemble of values) is made through a beamforming algorithm. There are various known beamforming algorithms. Although only one is described herein, the method may be adapted to other beamforming algorithms.

In a beamforming algorithm, the time lag between the transmissions of the excitation waves 21 by each transducer 26 is compensated. When the excitation waves 21 are transmitted in a medium considered homogenous, the speed of sound in that medium is usually taken into account. In general, a known non-measured standard speed of sound is chosen as a parameter. As a consequence, in a nonhomogenous medium, a known non-measured standard speed of sound is only an approximation of the real speed of sound in the medium.

The method described herein proposes to compensate for the inhomogeneity of the medium in order to approximate more accurately the speed of sound of the target region 12. It is indeed desirable to have a precise estimate of the speed of sound, in particular in a liver example, because a difference between normal and sick liver speed of sound is quite narrow.

Below an example of beamforming calculation in the case of planar waves to be transmitted into a homogeneous medium is given. To transmit a plane wave in a medium, the following delays are to be applied to the transducers in the probe:

$$d(i)=i.p$$

where

i is an index for a transducer in the probe, between 1 to N, N being the number of transducers, and
p is a predetermined time length in seconds.

Such delays are applied to transmit ultrasound pulses by the transducers in the medium.

Then, the plane wave is inclined at the penetration angle $\alpha$ with respect to the surface direction D1, and:

$$\sin(\alpha) = p.c0/\Delta x,$$

where

c0 is the constant reference speed of sound in the medium,
$\Delta x$ is the spatial pitch of the transducers in the probe (linear probe array).

Therefore, the penetration angle $\alpha$ can be recomputed for any speed of sound.

Then, the time needed for an emitted excitation wave 21 to reach a location in the medium 14 is known as "forward delay $D_{forward}$" and is defined as follows:

$$D_{forward} = 1/c0.[x.\sin(\alpha(c0))+z.\cos(\alpha(c0))] \quad \textbf{(Eq. 1)}$$

where:

$\alpha$ is at the penetration angle $\alpha$ with respect to the surface direction D1 of the planar excitation wave 21
x, z are the abscises along direction D1 and depth direction D of the location, and
c0 is the constant reference speed of sound in the medium, and
sin(), cos() are the sinus and cosinus trigonometric mathematical functions.

The time needed for the backscattered wave 29 to travel back to a given transducer at position xp is known as "backward delay $D_{backward}$" and is defined as:

$$D_{backward} = 1/c0.sqrt((x-xp)^2+z^2) \quad \textbf{(Eq. 2)}$$

where:
sqrt() is the square root mathematical function.

The total travel time D for a steered planar wave to reach the location and to travel back is thus:

$$D = D_{forward} + D_{backward} \quad \textbf{(Eq. 3)}$$

A simplified version of the beamforming algorithm is firstly to sum the sensed signal from all transducers of index i, phased by the above total travel time:

$$S_{coherent} = \sum_{i=1}^{N}[b(i).S_i(t+D)]$$

(Eq. 4)

where

b(i) is a transducers weighting function of the sensed signals, and

$S_i(t)$ is the sensed signal received by the transducer of index i corresponding to a position xp.

Thereafter, the beamforming algorithm calculates the energy in the coherent signal $S_{coherent}$ for a time window to deduce a value of pixel having coordinates (x, z) in the image data.

The method 40 for determining the target speed of sound cT comprises a step of beamforming image data on each of the intermediate region 30 and the target region 12 to obtain image data used in the calculation of a local speed of sound in the intermediate region 30 and the target region 12. The beamforming algorithm for the target region 12 uses the speed of sound previously determined using the beamforming algorithm for the intermediate region 30.

The method 40 comprises at least the following steps:

- At step S1, a virtual segmentation is realized. A position of the interface 34 between intermediate region 30 (e.g. muscle and connective tissue) of the medium 14 and the target region 12 (e.g. liver) in the depth direction D is determined on a morphological image of the medium. This determination will be used to apply different speed of sounds in the beamforming algorithm to the different identified regions.

The determination of the position of the interface 34 can be based on a morphological image of the medium 14. The morphological image can be:

- some of the sensed signals received by the probe 20, or
- an image data obtained by a beamforming algorithm processing the sensed signals, such as a B-mode image, or
- a recorded image stored previously in the ultrasound imaging system, such as an ultrasound image previously recorded by the system or from another system, or such as a magnetic resonance image (MRI) previously recorded from an magnetic resonance imaging system, or
- any other image corresponding to the medium 14.

The determination of the position of the interface 34 can be based on some of the sensed signals. A beamforming algorithm may be used to obtain image data of the medium 14 globally. These image data may then be processed to obtain the visual image 25 shown in Figure 2. The image data are exploited to determine the position of the interface 34. Example of determination of the interface 34 using the image data will be provided below.

At step S2, a plurality of first image data associated with a **first representative area 42** of the intermediate region 30 is calculated, and a **first speed of sound c1** of the intermediate region 30 based on the plurality of first image data is determined. The first representative area 42 is chosen in one embodiment proximal to the interface 34. The first representative area 42 is typically a sub-area of the intermediate region 30. The first representative area 42 is taken as representative of the intermediate region 30, in so far as the speed of sound that is determined using the plurality of first image data is considered to be the speed of sound for the intermediate region 30, herein referred as the first speed of sound c1. Each of the first image data is determined based on a beamforming algorithm applied to at least the sensed signals corresponding to the first representative area 42 and which takes as a parameter a **first reference speed of sound c_ref_1,** and one of a plurality of **first supposed speeds of sound c_supp_1.**

The first reference speed of sound c_ref_1 is for example a known speed of sound for the intermediate region 30.

For upcoming computations, when the speed of sound inside the intermediate region 30 is used as a parameter, the value of the determined first speed of sound c1 is used.

- At step S3, a plurality of target image data associated with a target representative area 44 of the target region 12 is calculated, and the target speed of sound cT inside the target region 12 based on the plurality of target image data is determined. The target representative area 44 may be selected in a similar way to the one described above for the first representative area 42. Each of the target image data is determined based on a beamforming algorithm (which may be the same beamforming algorithm as used above or another one) applied to at least the sensed signals corresponding to the target representative area 44 and which takes as parameters the position of the interface 34, a **second reference speed of sound c_ref_2** applied to the target region 12, the first speed of sound c1 applied to the intermediate region 30, and one of a plurality of **second supposed speeds of sound c_supp_2** for the target region 12.

### *Ways* to determine *the position of the* interface

In one embodiment, the position of the interface 34 is found by analyzing a gradient of the image data in the depth direction D. Because the density of the intermediate region 30 is substantially different from the one of the target region 12 (e.g. fatty tissues vs. liver), by analyzing the variation of the values of the image data in the depth direction D (and optionally in the surface direction D1) one may be able to determine, preferably automatically, the transition from the intermediate region 30 to the target region 12.

Thus, in one embodiment, the position of the interface 34 is determined based on variations of amplitudes of image data of the medium 14 along the depth direction D between the intermediate region 30 and the target region 12. For example, the variations of amplitudes of image data of the medium 14 along the depth direction D can be fitted to a parametric curve defining known evolution in such medium by any optimization algorithm. The parametric curve comprises several parameters, including for example the position of the interface 34 and other parameters.

The image data may be in the form of a 2D matrix, and is determined based on the sensed signals and a beamforming algorithm which takes as a parameter a reference speed of sound c0. The reference speed of sound c0 may correspond to a typical value of the speed of sound for the medium 14, i.e. a global reference speed of sound c_ref_0.

In one embodiment, the position of the interface 34 is determined by an automatic image processing of the morphological image or any 2D image data corresponding to the medium.

The automatic image processing may use a shape recognizing algorithm to determine the position of the interface 34.

The automatic image processing may use a conventional contour detection filter to determine the position of the interface 34. An example of such filter is a Canny or a Gabor filter.

Other ways to determine the position of the interface 34 are contemplated. For example, image data are processed by a neural network that is trained with a plurality of classified image data of similar medium, said classified image data being indexed with physical characteristics of the medium, and said physical characteristics including the position of the interface in each of said classified image data. The above processing may be a deep learning method based on hundreds or thousands of classified image data.

In another embodiment, the position of the interface 34 could be arbitrarily chosen. The arbitrary choice of the position of the interface 34 could be made in function of known values of depth of the intermediate region 30. For example, it is standard that in a human body a layer of skin and fatty cell be 2 cm thick. In such case the position of the interface 34 will be chosen to be at a distance of 2 cm in the depth direction D relative to the outer surface 26.

In yet another embodiment, the position of the interface is chosen so that a ballistic echo is strong between the two regions separated by the interface 34. In other words, the position of the interface 34 is determined at an instant of time $t_i$ when a sensed signal or the phased sensed signals (beamformed signal) is maximum.

The position of the interface may be solely a coordinate in the depth direction D, or coordinates in the depth direction D and the surface direction D1.

According to the above methods, the position of the interface 34 is preferably automatically determined in at least one of image data. Eventually, several image data are used (image data taken at various time instants), and the plurality of the determined positions are used to determine a best position, by for example a mean value of the successive determined positions of the interface 34.

### *Choice of the first representative* area

More specifically, referring to step S2, the first representative area 42 is chosen automatically by the processing unit 16 following a representative area selection algorithm. The first representative area 42 may be chosen to be a fixed portion of the intermediate region 30. For example, the first representative area 42 may be chosen to be a rectangle of 1 cm width in the depth direction D and 2 cm long in the surface direction D1.

Considering that the abdominal portion of the body has a muscle and connective tissues generally between 1 cm and 3 cm thick in the depth direction D, it may be advantageous to program the processing unit 16 to consider the first representative area 42 to be a rectangle of the visual image 25 located at, for example, 2 cm in the depth direction, and be midway of the image in the surface direction D1. In other embodiments, the representative area 42 could be chosen in function of the image data globally. For example a quasi-homogeneous area of the intermediate region 30 may be chosen.

Advantageously, the automatic selection allows obtaining a same result for the same data input. Additionally, the above steps may be performed once the patient data are collected, remotely in time and/or distance from the data collection, without need for his or her presence for additional scans.

### Obtaining first image data

The determination of the first image data is based on at least some of the sensed signals and a beamforming algorithm, such as the one described above, and which takes as a parameter the first reference speed of sound c_ref_1. The first reference speed of sound c_ref_1 is a constant parameter which may be retrieved from a memory of the processing unit 16 and may correspond to classical values of the speed of sound in the medium 14. This value is independent from the sensed signals.

For example, a beamforming calculation in the case of planar waves transmitted into a homogeneous medium in the first representative area 42 in the intermediate region 30 can be calculated by the following formula:

$$\texttt{D}_{\texttt{forward}} \texttt{ = (1/c1).[x.sin}(\alpha\texttt{(c1))+z.cos}(\alpha\texttt{(c1))]} \quad \textbf{(Eq. 5)}$$

$$\texttt{D}_{\texttt{backward}} \texttt{ = (1/c1).sqrt((x-xp)}^2\texttt{+z}^2\texttt{)} \quad \textbf{(Eq. 6)}$$

$$\texttt{D = D}_{\texttt{forward}} \texttt{ + D}_{\texttt{backward}} \quad \textbf{(Eq. 7)}$$

where:

$D_{forward}$ is the forward delay as explained above in a general case,
$D_{backward}$ is the backwark delay as explained above in a general case, and
c1 is the speed of sound to be determined in the medium of the first representative area 42 in the intermediate region 30,
these formula being corrected by the following formula:

$$\texttt{z = h(c\_ref\_1).c1/c\_ref\_1} \quad \textbf{(Eq. 8)}$$

$$\alpha\texttt{(c1) = asin( c1/c\_ref\_1.}\alpha\texttt{(c\_ref\_1))} \quad \textbf{(Eq. 9)}$$

where:

asin() is the arcsinus trigonometric mathematical function, and
h(c_ref_1) is a depth reference of the first representative area 42.

These corrections are to compensate the variation of speed of sound among the first supposed speeds of sound c_supp_1, so as first representative area 42 does not move in the intermediate region 30 and so as the excitation waves as plane waves have the same penetration angle $\alpha$ with respect to the surface direction D1. Thanks to these corrections, the produced first image data can be compared one to the other, and processed to calculate the first speed of sound c1 as explained.

Then, the beamforming algorithm sums the sensed signals $S_i$ from all transducers of index i, phased by the above total travel time:

$$S_{coherent} = \sum_{i=1}^{N}[b(i).S_i(t + D)]$$

$$\textbf{(Eq. 10)}$$

and calculates the energy in the coherent signal $S_{coherent}$ for a time window to deduce a value of pixel having coordinates (x, z) in the first image data.

The first reference speed of sound c_ref_1 may correspond to a known value of the speed of sound globally in the medium 14. It may be equal to the reference speed of sound c0. The first reference speed of sound c_ref_1 may correspond to a known value of the speed of sound solely in the intermediate region 30. For example, it is known that the speed of sound in fatty tissues is about 1480 m/s. The first reference speed of sound c_ref_1 may alternatively correspond to a known value of the speed of sound solely in the region of interest 12. It is known that the speed of sound in the liver can be for example about 1540 m/s.

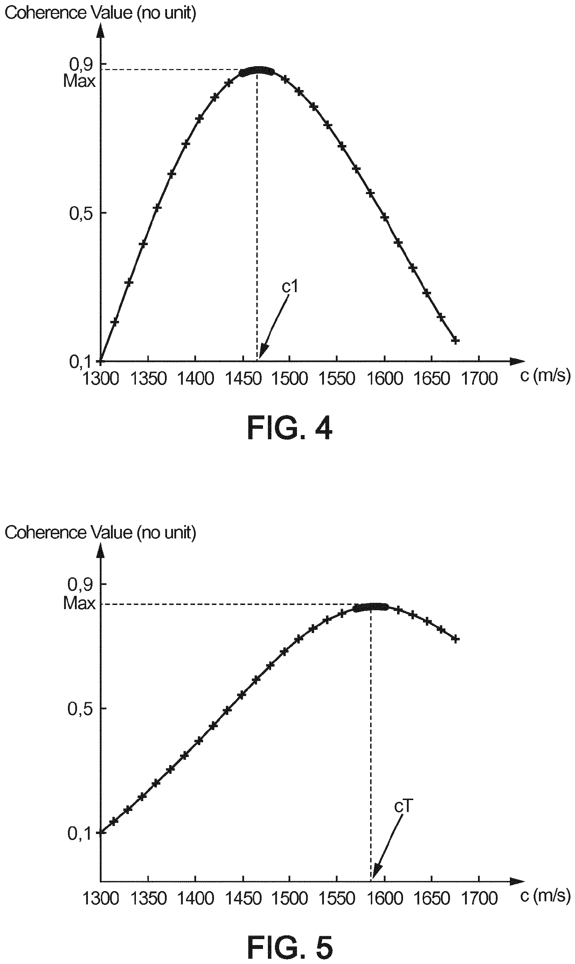

The plurality of first supposed speeds of sound c_supp_1 may be various known values of the speed of sound in the medium 14 (e.g. medium 14 globally or intermediate region 30, or the target region 12) which form a range of possible referenced speeds of sound. The range of possible referenced speeds of sound can be a range between 1300 m/s and 1700 m/s, as shown on the abscissa of **figure 4.** The first reference speed of sound c_ref_1 may be one of the plurality of first supposed speeds of sound c_supp_1.

### *Computation of the first speed of sound*

The first speed of sound c1 is calculated using a first focusing criterion. A plurality of first focusing values is obtained by applying the first focusing criterion to each of the plurality of first image data of the first representative area 42. The first speed of sound c1 is then a selected one of the plurality of respective first focusing values (i.e. the speed of sound corresponding to a selected first focusing value). In one embodiment, the first speed of sound c1 is the maximum of the plurality of respective first focusing values.

Should the first focusing criterion include a coherence criterion, the first speed of sound c1 would be chosen as the maximum of the plurality of values obtained using the first coherence criterion applied to the plurality of first image data, i.e. for each supposed first speed of sound c_supp_1. Figure 4 shows an example of coherence values obtained using the plurality of first speed of sound c_supp_1 (the coherence values being each point of the graph, with the values on the ordinate axis and the corresponding supposed speed of sound used to obtain the coherence values being on the abscissa axis). The first speed of sound c1 is selected as the speed of sound corresponding to the maximum of the coherence values calculated. In the example of the figure 4, the maximum is the highest point of the bell shape graph, which is about 2.47 on the ordinate axis, and which corresponds to a speed of sound of 1460 m/s.

In another embodiment, the focusing criterion includes a resolution criterion, such as a maximum lateral resolution. The resolution of an ultrasonic image is improved when the beamforming focusing delays are accurately compensating the propagation time of flights. Therefore, using an estimation of the resolution in an ultrasound image (image data) can be used as a focusing criterion for estimating the speed of sound in the medium.

In a first example, a Fourier transform on image data corresponding to a vertical or lateral line of the first image data (i.e. a line having a constant z coordinate) is used. Then, the first focusing criterion can be the frequency bandwidth of frequency values obtained by the Fourier transform. Similarly, the first speed of sound c1 would be chosen as the maximum of the plurality of values obtained using the first focusing criterion applied to the plurality of first image data, i.e. for each supposed first speed of sound c_supp_1. In other words, the first speed of sound c1 is chosen as the speed of sound that provides the greater frequency bandwidth for the frequency values obtained by the Fourier transform. This corresponds to having the best (maximum) resolution in the plurality of first image data.

In a second example, the resolution criterion is estimated by calculating a spatial autocorrelation of image data, and by estimating the size of spatial autocorrelation.

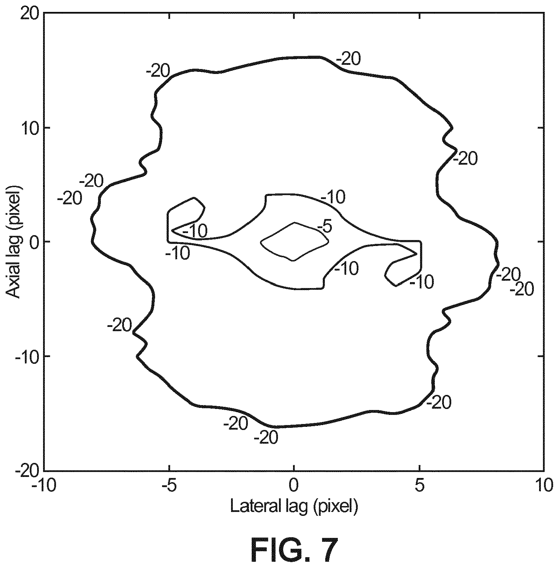

Figure 7 shows an example of spatial autocorrelation of two dimension (2D) image data. This spatial autocorrelation is also a 2D surface having a peak at the center, i.e. coordinate (0, 0) in an axial and lateral lag. In this figure, the spatial autocorrelation is represented as a plurality of level contour curves.

Then reasoning in each direction, axial and lateral, a peak width can be derived in each of said direction. The width can be defined as the width of the peak shape curve for a height at half of the peak maximum (i.e. width at -6 dB). Then, the size of the spatial autocorrelation is a function of said widths in each direction, and for example, it can be the maximum or mean value of the widths or determined by any other modulus function.

The focusing criterion may include more than one criterion. For example, the focusing criterion could include a coherence criterion and a maximum resolution criterion.

Other models of focusing criterion are contemplated.

### *Choice of the target representative area*

More specifically, referring to step S3, the target representative area 44 is chosen automatically by the processing unit 16 in a similar way to the first representative area, yet applied to the target region 12. The target representative area 44 may be chosen to be a fixed portion of the target region 12. For example, the target representative area 44 may be chosen to be a rectangle of 1 cm wide in the depth direction D and 2 cm long in the surface direction D1. The size and shape of the target representative area 44 may be different or the same as the one of the first representative area 42.

The target representative area 44 may also be chosen to be at a fixed position on the visual image 25. Considering that the abdominal portion of the body has a muscle and connective tissues generally between 1 cm and 3 cm thick in the depth direction D, on human beings it may be advantageous to program the processing unit 16 to consider the target representative area 44 to be a rectangle of the visual image 25 located at, for example, 7 cm in the depth direction, and be midway of the image in the surface direction D1. In other embodiments, the target representative area 44 could be chosen in function of the image data globally. For example a quasi-homogeneous area of the target region 12 may be considered.

### *Obtaining target image data*

The determination of the target image data that are associated with the target representative area 44 is based on at least some of the sensed signals and a beamforming algorithm, such as the one described above, and which takes as a parameter the target reference speed of sound c_ref_tar, the position of the interface 34, the first speed of sound c1 and a plurality of supposed target speeds of sound c_supp_tar.

The target reference speed of sound c_ref_tar is for example a known speed of sound for the target region 12.

The target reference speed of sound c_ref_tar is a constant parameter which may be retrieved from the memory of the processing unit 16 and which may correspond to classical values of the speed of sound in the medium 14. This value is independent from the sensed signals.

For example, a beamforming calculation in the case of planar waves transmitted into a homogeneous medium in the target representative area 44 in the target region 12 can be calculated by the following formula:

$$\mathrm{D_{forward}} = \texttt{(1/c1).[h1/cos(\alpha(c1))]} +$$
$$\texttt{(1/c).[(x-h1.tan(\alpha(c1)).sin(\alpha(c1))) + (z-h1).cos(\alpha(c))]}$$

$$\textbf{(Eq. 11)}$$

$$\mathrm{D_{backward}} = \texttt{min}_{\texttt{xi}}\texttt{[ (1/c1).sqrt((xi-xp)}^{2}\texttt{+h}^{2}\texttt{)} +$$
$$\texttt{(1/c2).sqrt((x-xi)}^{2}\texttt{+(z-h1)}^{2}\texttt{) ]} \qquad \textbf{(Eq. 12)}$$

$$\mathrm{D = D_{forward} + D_{backward}} \qquad\qquad \textbf{(Eq. 13)}$$

where:

$D_{forward}$ is the forward delay a,
$D_{backward}$ is the backward delay,
h1 is the thickness of the intermediate region 30 (i.e. the position of the interface 34),
c1 is the speed of sound previously determined in in the intermediate region 30,
c is the speed of sound to be determined in the medium of the target representative area 44 in the target region 12, these formula being corrected by the following formula:

$$\texttt{z = z\_ref.c/c\_ref\_2 + h1.(1-c/ c\_ref\_2)} \quad \textbf{(Eq. 14)}$$

where
z_ref is a depth reference of the target area 44.

This correction aims to compensate the variation of speed of sound among the supposed target speeds of sound c_supp_tar, so as target representative area 44 does not moves in the target region 12. Thanks to this, the produced target image data can be compared one to the other, and processed to calculate the target speed of sound c as explained above.

Then, the beamforming algorithm sums the sensed signals $S_i$ from all transducers of index i, phased by the above total travel time:

$$S_{coherent} = \sum_{i=1}^{N}[b(i).S_i(t+D)]$$

$$\textbf{(Eq. 15)}$$

and calculates the energy in the coherent signal $S_{coherent}$ for a time window to deduce a value of pixel having coordinates (x, z) in the target image data.

The target reference speed of sound c_ref_tar may correspond to a known value of the average speed of sound globally in the medium 14 considered being homogenous. It may be set equal to the reference speed of sound c0 and/or to the first reference speed of sound c_ref_1. The target reference speed of sound c_ref_tar may correspond to a known value of the speed of sound solely in the intermediate region 30. For example, it is known that the speed of sound in fatty tissues is about 1480 m/s. The target reference speed of sound c_ref_tar may alternatively correspond to a known value of the speed of sound solely in the region of interest 12. For example, it is known that the speed of sound in the liver is about 1540 m/s.

The plurality of supposed target speeds of sound c_supp_tar are various known values of the speed of sound (e.g. medium 14 globally, intermediate region 30, or target region 12) which form a range of possible reference speeds of sound. The plurality of supposed target speeds of sound c_supp_tar may be the same as the plurality of first supposed speeds of sound c_supp_1. The target reference speed of sound c_ref_tar may be one of the plurality of supposed target speeds of sound c_supp_tar.

### Computation of the target speed of sound

The target speed of sound cT is calculated using a focusing criterion similarly to the first speed of sound c1. As such, the detailed description of this step is very similar to that of the first step. A plurality of second focusing values is obtained by applying a target focusing criterion to each of the plurality of target image data of the target representative area 44. The target focusing criterion may be same or different as the first focusing criterion. As mentioned above, the focusing criterion may include a coherence criterion and/or a maximum lateral resolution criterion.

Referring to **Figure 5,** an example of graph of the coherence values obtained for different supposed speed of sound using the target image data is provided.

Each point of the graph corresponds to a coherence value (ordinate axis) obtained using one of the plurality of supposed speed of sound (abscissa axis) in the beamforming algorithm to obtain the first image data. The target speed of sound cT is selected as the speed of sound corresponding to the maximum of the plurality of coherence values calculated. In the example of the figure 5, the maximum is the highest point of the bell shape graph, which is about 0.55 on the ordinate axis, and which corresponding to a speed of sound of 1585 m/s.

### Adding aberration correction

The beamforming algorithm can be refined by taking into account aberration delays $D_{aberration}$. These aberration delays $D_{aberration}$ are arbitrary time of flights that are not due to the propagation of waves in the medium, and that are not taken into account by the propagation model, and therefore cannot be compensated by adjusting the parameters of the model such as the global speed of sound if an homogeneous model is chosen. These aberration delays can compensate delays in each physical transducers that can be slightly different one to another, delays in the electronic for signal amplifications, and delays in material layers in the probe.

Then, the formula of total travel time D for the beamforming can be modified as:

$$D = D_{forward} + D_{backward} + D_{aberration},$$

which permits to improve the phasing of sensed signals in the beamforming sum of equations 4, 10 and 15.

The document "Robust sound speed estimation for ultrasound-based hepatic steatosis assessment", Marion Imbault et Al gives an example of such refinement. However, such aberration correction cannot correctly and accurately compensate the propagation of speed of sound in medium that is inhomogeneous, i.e. having modifications of speed of sound during the wave propagation in the medium.

On the contrary, the method disclosed herewith determines at least a first speed of sound c1 and a target speed of sound cT. The method therefore builds a model of the medium having a plurality of speed of sounds (at least two) that minimize the amplitude of $D_{aberration}$ and therefore leads to a much more accurate estimation of the target speed of sound cT, which can very important in medical application, such as evaluation of hepatic steatosis or other disease.

### Case of three regions

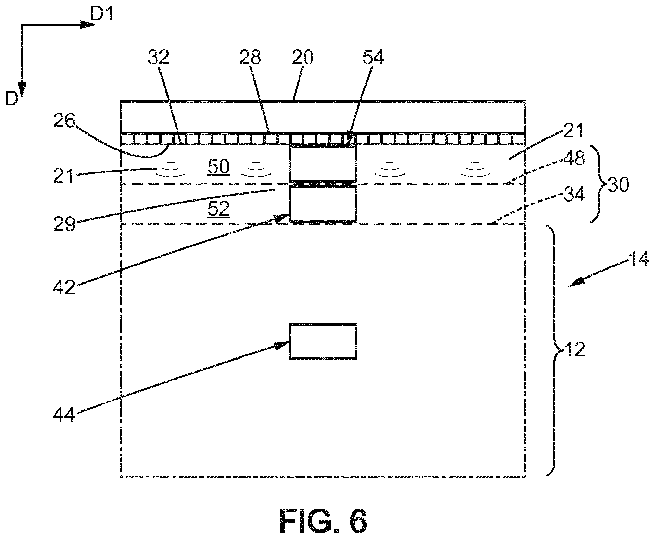

Referring now to **Figure 6,** the above could be generalized to 3 or more stacked regions. The segmentation in finer regions of the intermediate region 30 would increase the precision of the value calculated for the target speed of sound cT.

The intermediate region 30 may be segmented in one embodiment into two regions. A **sub-interface 48** divides in the depth direction D the intermediate region 30 into a **second region 50** of the intermediate region 30 proximal to the outer surface 26 and containing a second representative area 54 and a **first region 52** of the intermediate region 30 proximal to the interface 34 and containing the first representative area 42. The sub-interface 48 could, for example, correspond to an interface between a skin layer and a muscle layer.

The target speed of sound cT may then be determined based on the first speed of sound c1 as previously described, but the first speed of sound c1 would be determined based on a second speed of sound c2 of the second region 50. Thus, the target speed of sound cT in a medium segmented into a plurality of stacked region is calculated via the successive determination of adjacent stacked regions, from the most distal region to the target region 12, to the most proximal region to the target region 12.

The method to determine the target speed of sound cT using a segmentation into three regions may be as follows:

- Step 10: determining a position of the sub-interface 48 in the intermediate region 30 and the interface in the medium 14, the determination of the position of the sub-interface 48 and the position of the interface 34 being based on at least some of the sensed signals. The determination of the position of the interface 34 could be delayed until after step 11 and before step 12.
- Step 11: calculating a plurality of second image data associated with a **second representative area 54** of the second region 50 and determining a **second speed of sound c2** of the second region 50 based on the plurality of second image data. The second representative area 54 may be determined in a similar way as the first representative area 42. Each of the second image data is determined based on a beamforming algorithm applied to at least the sensed signals corresponding to the second representative area 54 and which takes as a parameter a **second reference speed of sound c_ref_2,** and one of a plurality of **second supposed speeds of sound c_supp_2,** The second reference speed of sound c_ref_2 may be a reference speed for the type of tissue found in the second region 50. For example, it might be a standard speed of sound in epidermis type of tissues. The second supposed speed of sound c_supp_2 may be traditional expected speeds of sound for that type of tissue.

- Step 12: calculating a plurality of first image data associated with the first representative area 42 of the intermediate region 30 and determining the first speed of sound c1 of the intermediate region 30 based on the plurality of first image data. The first representative area 42 is in one embodiment proximal to the interface 34 in the second region 52. Each of the first image data is determined based on a beamforming algorithm applied to at least the sensed signals corresponding to the first representative area 42 and which takes as a parameter a position of the sub-interface 48, the second speed of sound c2, a first reference speed of sound c_ref_1, and one of a plurality of first supposed speeds of sound c_supp_1. The first reference speed of sound c_ref_1 may be a reference speed for the type of tissue found in the first region 52 or in the intermediate region 30 globally, as previously used. For example, it might be a standard speed of sound in muscles. The first supposed speed of sound c_supp_1 may be traditional expected speeds of sound for that type of tissue. The first reference speed of sound c_ref_1 and/or the first supposed speeds of sound c_supp_1 may or may not be respectively equal to the second reference speed of sound c_ref_2 and/or the second supposed speeds of sound c_supp_2.
- Step 13: calculating the plurality of target image data associated with the target representative area 44 of the target region 12 and determining the target speed of sound cT inside the target region 12, based on the plurality of target image data in the representative area 44 of the target region 12. Each of the target image data is determined based on a beamforming algorithm applied to at least the sensed signals corresponding to the representative area 44 and which takes as parameters the position of the interface 34, the target reference speed of sound c_ref_tar applied to the target region 12, the first speed of sound c1 applied to the intermediate region 30, and one of a plurality of supposed target speeds of sound c_supp_tar for the target region 12.

A method for determining the target speed of sound cT in the target region 12, according to **a second embodiment,** will be now described. This embodiment does not use the calculation of image data in representative areas. Instead the raw sensed signals $S_i$ are directly used for one predetermined location in the medium in each representative area. This embodiment may advantageously reduce the calculations to obtain the first and target speeds of sound.

Similarly to the first embodiment of the method 40, the medium 14 is segmented into the intermediate region 30 and the target region 12, using one of the ways described above.

Following the segmentation of the medium 14, in a first step, the first speed of sound c1 of the intermediate region 30 is determined directly based on at least some of the sensed signals. The determination of the first speed of sound c1 takes into account the first reference speed of sound c_ref_1, and one of the plurality of first supposed speeds of sound c_supp_1.

One can calculate a first focusing criterion as the following ratio C:

$$C = \frac{\langle |S(t)|^2 \rangle}{N \cdot \Sigma_{i=1}^{N} \langle |S_i(t - \tau_i)|^2 \rangle}$$

(Eq. 16)

with

$$\langle |S(t)|^2 \rangle = \left\langle \left| \sum_{i=1}^{N} S_i(t - \tau_i) \right|^2 \right\rangle$$

(Eq. 17)

where:

$S_i$ is a sensed signal for transducer of index i,
N is the number of transducers,
$\tau_i$ is the total travel time D for waves to reach a predetermined focused location and to travel back to a transducer, as defined above, and
the brackets <> represents a time window average.

Therefore, the ratio C is a ratio between the coherent intensity and total incoherent intensity, and it satisfies the following inequality:

$$0 \leq C \leq 1$$

This ratio C therefore represents a quality of the focusing functions that are applied for calculating the total travel of time, and it can be used as a first focusing criterion. The ratio C is then maximum (as represented on figure 4) if the first supposed speeds of sound c_supp_1 is the related to the best focusing functions, i.e. if the first supposed speeds of sound c_supp_1 is close to (ideally equal to) the first speed of sound c1.

In a second step, the target speed of sound cT is also determined inside the target region 12 directly based on at least some of the sensed signals. The determination of the target speed of sound cT takes into account the position of the interface 34, the target reference speed of sound c_ref_tar applied to the target region 12, the first speed of sound c1 applied to the intermediate region 30, and one of the plurality of supposed target speeds of sound c_supp_tar for the target region 12.

The target focusing criterion is computed with the ratio C. This ratio C now takes into account the interface 34 and the first speed of sound c1 in the parameter $\tau_i$ that is the total travel time D for waves to reach the predetermined focused location and to travel back to a transducer. It is used as the target focusing criterion, and it has a maximum (as represented on figure 5) if the supposed target speed of sound is close to (ideally equal to) the target speed of sound cT.

A variant of the above second embodiment is now explained. In the second embodiment, the ratio C considers sensed signals and summed of sensed signals that are time averaged via the brackets <> mathematical function, and that the ratio is calculated for a predetermined focused location (x, z) in the medium.

In the variant, the time averaging is replaced by an averaging over a plurality of focused locations. Then, the first focusing criterion is calculated via the following spatial ratio Cs:

$$Cs = \frac{\Sigma_{Mj} \left( \left| \Sigma_{i=1}^{N} S_i(t - \tau_{i,Mj}) \right|^2 \right)}{N \cdot \Sigma_{Mj} \left( \Sigma_{i=1}^{N} \left| S_i(t - \tau_{i,Mj}) \right|^2 \right)}$$

(Eq. 18)

where

$S_i$ is a sensed signal for transducer of index i,

16

N is the number of transducers,

$\tau_{i,Mj}$ is the total travel time D for waves to reach a focused location Mj and to travel back to a transducer i, as defined above, and

Mj is a location in the plurality of focused locations in the medium.

A method for determining the target speed of sound cT in the target region 12, according to a **third embodiment,** will be now described. This embodiment does not use the calculation of image data in representative areas. Instead the raw sensed signals Si are directly used for one predetermined focused location in the medium in each representative area. This embodiment may also reduce the calculations to obtain the first and target speeds of sound.

Similarly to the first embodiment of the method 40, the medium 14 is segmented into the intermediate region 30 and the target region 12, using one of the ways described above.

Following the segmentation of the medium 14, in a first step, the first speed of sound c1 of the intermediate region 30 is determined directly based on at least some of the sensed signals. The determination of the first speed of sound c1 takes into account the first reference speed of sound c_ref_1, and one of the plurality of first supposed speeds of sound c_supp_1.

Ones can calculate the following spatial covariance R thanks to the following formula:

$$\hat{R}(n) = \frac{N}{N-n} \cdot \frac{\sum_{i=1}^{N-n}(\int_{t_f-\frac{T}{2}}^{t_f+\frac{T}{2}} S_i(t-\tau_i).S_{i+n}(t-\tau_i).dt)}{\sum_{i=1}^{N}(\int_{t_f-\frac{T}{2}}^{t_f+\frac{T}{2}} S_i^2(t-\tau_i).dt)}$$

$$\text{(Eq. 19)}$$

where

$S_i(t)$ is the sensed signal for the transducer i,

n is an index distance to the transducer i,

N is the number of transducers,

$t_f$ is the focal time at the predetermined focused location,

$\tau_i$ is the total travel time D for waves to reach a predetermined focused location and to travel back to a transducer, as defined above, and

T is a time window length for the analysis.

Such transducers spatial covariance versus the transducer distance n is equal to 1 for n=0, and decreases with the increase of distance n. Thus, its decreasing curve is ideally a triangle having a maximum at n=0 if focusing at the predetermined focused location is perfect: i.e. if the delay time law or estimation of forward and backwards time of flights are correct. This is the case, when the speed of sound used in these times of flights is correct. On the contrary, if the speed of sound used is not correct, transducers spatial covariance decrease more and its curve is more and more situated under the ideal triangle shape.

Consequently, the first focusing criterion can be estimated by an integral of the spatial covariance R over the distance index n, and this integral is maximum if the first supposed speeds of sound c_supp_1 is the related to the best focusing functions, i.e. if the first supposed speeds of sound c_supp_1 is close to (ideally equal to) the first speed of sound c1.

In a second step, the target speed of sound cT is also determined inside the target region 12 directly based on at least some of the sensed signals. The determination of the target speed of sound cT takes into account the position of the interface 34, the target reference speed of sound c_ref_tar applied to the target region 12, the first speed of sound c1 applied to the intermediate region 30, and one of the plurality of supposed target speeds of sound c_supp_tar for the target region 12.

The target focusing criterion is computed with the integral over distance index n of the above spatial covariance R. This calculation now takes into account the interface 34 and the first speed of sound c1 in the parameter the total travel time D for waves to reach the predetermined focused location and to travel back to the transducers. It is used as the target focusing criterion, and it has a maximum if the supposed target speed of sound is close to (ideally equal to) the target speed of sound cT.

Therefore, in all embodiments, the above method 40 for determining the target speed of sound inside a target region of a medium is using an ultrasound imaging system. The method comprises a steps of determining a position of an interface in the medium, determining a first speed of sound of an intermediate region above the interface, and determining a target speed of sound inside a target region below the interface based on at least some of sensed signals and taking into account the position of the interface and the first speed of sound.

This method and the ultrasound system implementing said method as described in the all above detailed description permits to build an inhomogeneous model of the medium and to accurately estimate a target speed of sound cT. The invention is defined in the appended claims.

**Claims**

1. **A method (40) for determining a target speed of sound (cT)** inside a target region (12) of a medium (14) using an ultrasound imaging system (10) comprising at least a probe (20) adapted to sense backscattered waves and to provide sensed signals corresponding to said backscattered waves to a processing unit (16) of the ultrasound system (10), the method (40) comprising:

   - determining on a morphological image a position of at least an interface (42) in the medium (14), the interface (34) dividing the medium (14) into an intermediate region (30) of the medium (14) and the target region (12) in a depth direction (D),
   - determining a first speed of sound (c1) of the intermediate region (30) based on at least some of the sensed signals), and
   - determining the target speed of sound (cT) inside the target region (12) based on at least some of the sensed signals and taking into account the position of the interface (34), and the first speed of sound (c1).

2. The method according to claim 1, wherein the probe (20) is adapted to be functionally put in contact with an outer surface (32) of the medium (14), the probe (20) being adapted to transmit excitation waves into the medium (14) in a depth direction (D) toward the target region (12), said excitation waves being backscattered in the medium (14) toward the probe (20), and

   wherein the first speed of sound (c1) is determined by taking into account a first reference speed of sound (c_ref_1), and one of a plurality of first supposed speeds of sound (c_supp_1), and
   wherein the target speed of sound (cT) is determined by taking into account the position of the interface (34), a target reference speed of sound (c_ref_tar) applied to the target region (12), the first speed of sound (c1) applied to the intermediate region (30), and one of a plurality of supposed target speeds of sound (c_supp_tar) for the target region (12).

3. The method according to claim 2, further comprising calculating a plurality of first image data associated with a first representative area (42) of the intermediate region (30) before determining the first speed of sound (c1) of the intermediate region (30), the first speed of sound being based on the plurality of first image data, each of the first image data being determined based on a beamforming algorithm applied to at least the sensed signals corresponding to the first representative area (42) and which takes as a parameter a first reference speed of sound (c_ref_1), and one of a plurality of first supposed speeds of sound (c_supp_1).

4. The method according to any one of claim 2 or 3, further comprising calculating a plurality of target image data associated with a target representative area (44) of the target region (12) before determining the target speed of sound (cT) inside the target region (12), the target speed of sound (cT) being based on the plurality of target image data in the representative area (44) of the target region (12), each of the target image data being determined based on a beamforming algorithm applied to at least the sensed signals corresponding to the representative area (44) and which takes as parameters the position of the interface (34), the target reference speed of sound (c_ref_tar) applied to the target region (12), the first speed of sound (c1) applied to the intermediate region (30), and one of the plurality of supposed target speeds of sound (c_supp_tar) for the target region (12).

5. The method according to any one of claims 1 to 4, wherein at least one of:

   - the plurality of first supposed speeds of sound (c_supp_1) and the plurality of supposed target speeds of sound (c_supp_tar) have a same value, and
   - the first supposed speeds of sound (c_supp_1) are known speeds of sound for the intermediate region (30), and the supposed target speeds of sound (c_supp_tar) are known speeds of sound for the target region (12).

6. The method according to any one of claims 1 to 5, wherein the determination of the position of the interface (34) is based on at least some of the sensed signals.

7. The method according to any one of claims 1 to 5, wherein the determination of the position of the interface (34) is based on an automatic image processing of the morphological image.

8. The method according to anyone of claims 1 to 6, wherein the position of the interface (34) is determined based on variations of amplitudes of image data of the medium (14) along the depth direction (D) between the intermediate region (30) and the target region (12), the image data being determined based on the sensed signals and a beamforming algorithm which takes as a parameter the reference speed of sound (c0).

9. The method according to anyone of claim 1 to 8, wherein the first speed of sound (c1) and/or the target speed of sound (cT) are each calculated using a respective first and/or target focusing criterion, a plurality of respective first and/or target focusing values being obtained by applying the respective first and/or target focusing criterion to, respectively, the plurality of first image data of the first representative area (42) and/or the plurality of target image data of the target representative area (44), the first speed of sound (c1) being a selected one of the plurality of respective first focusing values and/or the target speed of sound (cT) being a selected one of the plurality of respective target focusing values.

10. The method according to claim 9, wherein the first speed of sound (c1) is the maximum of the plurality of respective first focusing values and/or the target speed of sound (cT) is the maximum of the plurality of respective target focusing values.

11. The method according to claim 9 or 10, wherein the focusing criterion is a coherence criterion.

12. The method according to any one of the claims 1 to 11, further comprising:

- determining a position of a sub-interface in the intermediate region (30), the sub-interface dividing in the depth direction the intermediate region (30) into a second region of the intermediate region proximal to the outer surface and a first region of the intermediate region proximal to the interface (34),
- determining a second speed of sound (c2) of the second region based on at least some of the sensed signals and taking as a parameter a second reference speed of sound (c_ref_2), and one of a plurality of second supposed speeds of sound (c_supp_2),

wherein
the determination of the first speed of sound (c1) is based on at least some of the sensed signals and takes into account the position of the sub-interface, the first reference speed of sound (c_ref_1) applied to the first region, the second reference speed of sound (c_ref_2) applied to the second region, and one of the plurality of first supposed speeds of sound (c_supp_1) for the first region.

13. The method according to claim 12, wherein the determination of the position of the sub-interface being based on at least some of the sensed signals.

14. The method of any one of the claims 1 to 13, wherein the second region of the intermediate region contains the second representative area, and the first region of the intermediate region contains the first representative area.

15. The method of any one of the claims 1 to 14, further comprising calculating a plurality of second image data associated with a second representative area of the second region before determining the second speed of sound (c2) of the second region based on the plurality of second image data, each of the second image data being determined based on a beamforming algorithm applied to at least the sensed signals corresponding to the second representative area and which takes as a parameter a second reference speed of sound (c_ref_2), and one of a plurality of second supposed speeds of sound (c_supp_2).

16. The method according to any one of claims 1 to 15, wherein the medium (14) is a mammal body and the outer surface (26) is the skin of the mammal, and
wherein the target region (12) is the liver of the mammal, the intermediate region (30) is a region of the medium (14) comprised between the liver and the skin in the depth direction (D).

**17. An ultrasound imaging system (10) for determining a target speed of sound (cT)** inside a target region (12) of a medium (14), said ultrasound imaging system (10) comprising:

- a probe (20) adapted to be put functionally in contact with an outer surface (26) of the medium (14), the probe (20) being adapted to transmit excitation waves (21) into the medium (14) in a depth direction (D) toward the target region (12), said excitation waves (21) being backscattered in the medium (14) toward the probe (20), the probe (20) being adapted to sense the backscattered waves (29) and to provide corresponding sensed signals to the ultrasound system (10), and
- a processing unit (16) implementing the method according to any one of claims 1 to 16.

## Patentansprüche

1. Verfahren (40) zum Bestimmen einer Ziel-Schallgeschwindigkeit (cT) innerhalb einer Zielregion (12) eines Mediums (14) unter Verwendung eines Ultraschall-Bildgebungssystems (10), welches wenigstens eine Sonde (20) umfasst, welche dazu eingerichtet ist, rückgestreute Wellen zu erfassen und erfasste Signale, welche den rückgestreuten Wellen entsprechen, an eine Verarbeitungseinheit (16) des Ultraschall-Systems (10) bereitzustellen, wobei das Verfahren (40) umfasst:

   - Bestimmen an einem morphologischen Bild einer Position von wenigstens einer Grenzfläche (42) in dem Medium (14), wobei die Grenzfläche (34) das Medium (14) in eine Zwischenregion (30) des Mediums (14) und die Zielregion (12) in einer Tiefenrichtung (D) unterteilt,
   - Bestimmen einer ersten Schallgeschwindigkeit (c1) der Zwischenregion (30) auf Grundlage von wenigstens einigen der erfassten Signale, und
   - Bestimmen der Ziel-Schallgeschwindigkeit (cT) innerhalb der Zielregion (12) auf Grundlage von wenigstens einigen der erfassten Signale und unter Berücksichtigung der Position der Grenzfläche (34) und der ersten Schallgeschwindigkeit (c1).

2. Verfahren nach Anspruch 1, wobei die Sonde (20) dazu eingerichtet ist, funktional in Kontakt mit einer äußeren Fläche (32) des Mediums (14) gebracht zu sein, wobei die Sonde (20) dazu eingerichtet ist, Anregungswellen in das Medium (14) in einer Tiefenrichtung (D) in Richtung der Zielregion (12) zu übertragen, wobei die Anregungswellen in dem Medium (14) in Richtung der Sonde (20) zurückgestreut werden, und wobei die erste Schallgeschwindigkeit (c1) bestimmt wird, indem eine erste Referenz-Schallgeschwindigkeit (c_ref_1) und eine aus einer Mehrzahl von ersten vermeintlichen Schallgeschwindigkeiten (c_supp_1) berücksichtigt werden, und wobei die Ziel-Schallgeschwindigkeit (cT) durch Berücksichtigen der Position der Schnittstelle (34), einer Ziel-Referenz-Schallgeschwindigkeit (c_ref_tar), welche auf die Zielregion (12) angewendet wird, die erste Schallgeschwindigkeit (c1), welche auf die Zwischenregion (30) angewendet wird, und eine aus einer Mehrzahl von vermeintlichen Ziel-Schallgeschwindigkeiten (c_supp_tar) für die Zielregion (12) bestimmt wird.

3. Verfahren nach Anspruch 2, ferner umfassend ein Berechnen einer Mehrzahl von ersten Bilddaten, welche einem ersten repräsentativen Bereich (42) der Zwischenregion (30) zugeordnet sind, bevor die erste Schallgeschwindigkeit (c1) der Zwischenregion (30) bestimmt wird, wobei die erste Schallgeschwindigkeit auf der Mehrzahl von ersten Bilddaten basiert, wobei alle der ersten Bilddaten auf Grundlage eines Strahlformung-Algorithmus basieren, welcher auf wenigstens die erfassten Signale angewendet wird, welche dem ersten repräsentativen Bereich (42) entsprechen, und welcher als einen Parameter eine erste Referenz-Schallgeschwindigkeit (c_ref_1) und eine aus einer Mehrzahl von ersten vermeintlichen Schallgeschwindigkeiten (c_supp_1) nimmt.

4. Verfahren nach einem der Ansprüche 2 oder 3, ferner umfassend ein Berechnen einer Mehrzahl von Ziel-Bilddaten, welche einem repräsentativen Zielbereich (44) der Zielregion (12) zugeordnet sind, bevor die Ziel-Schallgeschwindigkeit (cT) innerhalb der Zielregion (12) bestimmt wird, wobei die Ziel-Schallgeschwindigkeit (cT) auf der Mehrzahl von Ziel-Bilddaten in dem repräsentativen Bereich (44) der Zielregion (12) basiert, wobei alle der Ziel-Bilddaten auf Grundlage eines Strahlformung-Algorithmus bestimmt werden, welcher auf wenigstens die erfassten Signale angewendet wird, welche dem repräsentativen Bereich (44) entsprechen, und welcher als Parameter die Position der Grenzfläche (34), die Ziel-Referenz-Schallgeschwindigkeit (c_ref_tar), welche auf die Zielregion (12) angewendet wird, die erste Schallgeschwindigkeit (c1), welche auf die Zwischenregion (30) angewendet wird, und eine aus der Mehrzahl von vermeintlichen Schallgeschwindigkeiten (c_supp_tar) für die Zielregion (12) nimmt.

5. Verfahren nach einem der Ansprüche 1 bis 4, wobei wenigstens eines gilt aus:

- die Mehrzahl von ersten vermeintlichen Schallgeschwindigkeiten (c_supp_1) und die Mehrzahl von vermeintlichen Ziel-Schallgeschwindigkeiten (c_supp_tar) weisen einen selben Wert auf, und
- die ersten vermeintlichen Schallgeschwindigkeiten (c_supp_1) sind bekannte Schallgeschwindigkeiten für die Zwischenregion (30) und die vermeintlichen Ziel-Schallgeschwindigkeiten (c_supp_tar) sind bekannte Schallgeschwindigkeiten für die Zielregion (12).

6. Verfahren nach einem der Ansprüche 1 bis 5, wobei die Bestimmung der Position der Grenzfläche (34) auf wenigstens einigen der erfassten Signalen basiert.

7. Verfahren nach einem der Ansprüche 1 bis 5, wobei die Bestimmung der Position der Grenzfläche (34) auf einer automatischen Bildverarbeitung des morphologischen Bilds basiert.

8. Verfahren nach einem der Ansprüche 1 bis 6, wobei die Position der Grenzfläche (34) auf Grundlage von Variationen von Amplituden von Bilddaten des Mediums (14) entlang der Tiefenrichtung (D) zwischen der Zwischenregion (30) und der Zielregion (12) bestimmt wird, wobei die Bilddaten auf Grundlage der erfassten Signale und einem Strahlformung-Algorithmus bestimmt werden, welcher als einen Parameter die Referenz-Schallgeschwindigkeit (c0) nimmt.

9. Verfahren nach einem der Ansprüche 1 bis 8, wobei die erste Schallgeschwindigkeit (c1) und/oder die Ziel-Schallgeschwindigkeit (cT) jeweils berechnet werden unter Verwendung eines jeweiligen ersten und/oder Ziel-Fokussierungskriteriums, wobei eine Mehrzahl von jeweiligen ersten und/oder Ziel-Fokussierungswerten erhalten wird, indem das jeweilige erste und/oder Ziel-Fokussierungskriterium auf jeweils die Mehrzahl von ersten Bilddaten des ersten repräsentativen Bereichs (42) und/oder die Mehrzahl von Ziel-Bilddaten des repräsentativen Zielbereichs (44) angewendet wird, wobei die erste Schallgeschwindigkeit (c1) ein ausgewählter aus der Mehrzahl der jeweiligen ersten Fokussierungswerte ist und/oder die Ziel-Schallgeschwindigkeit (cT) ein ausgewählter aus der Mehrzahl von jeweiligen Ziel-Fokussierungswerten ist.

10. Verfahren nach Anspruch 9, wobei die erste Schallgeschwindigkeit (c1) das Maximum der Mehrzahl von jeweiligen ersten Fokussierungswerten ist und/oder die Ziel-Schallgeschwindigkeit (cT) das Maximum der Mehrzahl von jeweiligen Ziel-Fokussierungswerten ist.

11. Verfahren nach Anspruch 9 oder 10, wobei das Fokussierungskriterium ein Kohärenzkriterium ist.

12. Verfahren nach einem der Ansprüche 1 bis 11, ferner umfassend:

- Bestimmen einer Position einer Unter-Grenzfläche in der Zwischenregion (30), wobei die Unter-Grenzfläche in der Tiefenrichtung die Zwischenregion (30) in eine zweite Region der Zwischenregion proximal zu der äußeren Fläche und eine erste Region der Zwischenregion proximal zu der Grenzfläche (34) unterteilt,
- Bestimmen einer zweiten Schallgeschwindigkeit (c2) der zweiten Region auf Grundlage von wenigstens einigen der erfassten Signale und wobei als ein Parameter eine zweite Referenz-Schallgeschwindigkeit (c_ref_2) und eine aus einer Mehrzahl von zweiten vermeintlichen Schallgeschwindigkeiten (c_supp_2) genommen wird,

wobei
die Bestimmung der ersten Schallgeschwindigkeit (c1) auf wenigstens einigen der erfassten Signale basiert und die Position der Unter-Grenzfläche, die erste Referenz-Schallgeschwindigkeit (c_ref_1), welche auf die erste Region angewendet wird, die zweite Referenz-Schallgeschwindigkeit (c_ref_2), welche auf die zweite Region angewendet wird, und eine aus der Mehrzahl von ersten vermeintlichen Schallgeschwindigkeiten (c_supp_1) für die erste Region berücksichtigt.

13. Verfahren nach Anspruch 12, wobei die Bestimmung der Position der Unter-Grenzfläche auf wenigstens einigen der erfassten Signale basiert.

14. Verfahren nach einem der Ansprüche 1 bis 13, wobei die zweite Region der Zwischenregion den zweiten repräsentativen Bereich enthält und die erste Region der Zwischenregion den ersten repräsentativen Bereich enthält.

15. Verfahren nach einem der Ansprüche 1 bis 14, ferner umfassend ein Berechnen einer Mehrzahl von zweiten Bilddaten, welche einem zweiten repräsentativen Bereich der zweiten Region zugeordnet sind, bevor die zweite Schallgeschwindigkeit (c2) der zweiten Region auf Grundlage der Mehrzahl von zweiten Bilddaten bestimmt wird, wobei

**EP 3 632 330 B1**

alle der zweiten Bilddaten auf Grundlage eines Strahlformung-Algorithmus bestimmt werden, welcher auf wenigstens die erfassten Signale angewendet wird, welche dem zweiten repräsentativen Bereich entsprechen, und welcher als einen Parameter eine zweite Referenz-Schallgeschwindigkeit (c_ref_2) und eine aus einer Mehrzahl von zweiten vermeintlichen Schallgeschwindigkeiten (c_supp_2) nimmt.

16. Verfahren nach einem der Ansprüche 1 bis 15, wobei das Medium (14) ein Säugetier-Körper ist und die äußere Fläche (26) die Haut des Säugetiers ist, und wobei die Zielregion (12) die Leber des Säugetiers ist, wobei die Zwischenregion (30) eine Region des Mediums (14) ist, welche zwischen der Leber und der Haut in der Tiefenrichtung (D) umfasst ist.

17. Ultraschall-Bildgebungssystem (10) zum Bestimmen einer Ziel-Schallgeschwindigkeit (cT) innerhalb einer Zielregion (12) eines Mediums (14), wobei das Ultraschall-Bildgebungssystem (10) umfasst:

- eine Sonde (20), welche dazu eingerichtet ist, funktional in Kontakt mit einer äußeren Fläche (26) des Mediums (14) gebracht zu sein, wobei die Sonde (20) dazu eingerichtet ist, Anregungswellen (21) in das Medium (14) in einer Tiefenrichtung (D) in Richtung der Zielregion (12) zu übertragen, wobei die Anregungswellen (21) in dem Medium (14) in Richtung der Sonde (20) rückgestreut werden, wobei die Sonde (20) dazu eingerichtet ist, die rückgestreuten Wellen (29) zu erfassen und entsprechende erfasste Signale an das Ultraschall-System (10) bereitzustellen, und
- eine Verarbeitungseinheit (16), welche das Verfahren nach einem der Ansprüche 1 bis 16 implementiert.

**Revendications**

1. Procédé (40) de détermination d'une vitesse cible du son (cT) à l'intérieur d'une région cible (12) d'un milieu (14) utilisant un système d'imagerie par ultrasons (10) comprenant au moins une sonde (20) apte à détecter des ondes rétrodiffractées et à fournir des signaux détectés correspondant auxdites ondes rétrodiffractées à une unité de traitement (16) du système ultrasonore (10), le procédé (40) comprenant :

- la détermination sur une image morphologique d'une position d'au moins une interface (42) dans le milieu (14), l'interface (34) divisant le milieu (14) en une région intermédiaire (30) du milieu (14) et de la région cible (12) dans une direction de profondeur (D),
- la détermination d'une première vitesse du son (c1) de la région intermédiaire (30) sur la base d'au moins une partie des signaux détectés, et
- la détermination de la vitesse cible du son (cT) à l'intérieur de la région cible (12) sur la base d'au moins une partie des signaux détectés et la prise en considération de la position de l'interface (34), et de la première vitesse du son (c1).

2. Procédé selon la revendication 1, dans lequel la sonde (20) est apte à être fonctionnellement mise en contact avec une surface externe (32) du milieu (14), la sonde (20) étant apte à transmettre des ondes d'excitation dans le milieu (14) dans une direction de profondeur (D) vers la région cible (12), lesdites ondes d'excitation étant rétrodiffractées dans le milieu (14) vers la sonde (20), et

dans lequel la première vitesse du son (c1) est déterminée en prenant en considération une première vitesse de référence du son (c ref 1), et l'une d'une pluralité de premières vitesses supposées du son (c_supp_1), et dans lequel la vitesse cible du son (cT) est déterminée en prenant en considération la position de l'interface (34), une vitesse de référence cible du son (c_ref_tar) appliquée à la région cible (12), la première vitesse du son (c1) appliquée à la région intermédiaire (30), et l'une d'une pluralité de vitesses cibles supposées du son (c_supp_tar) pour la région cible (12).

3. Procédé selon la revendication 2, comprenant en outre le calcul d'une pluralité de premières données d'image associées à une première zone représentative (42) de la région intermédiaire (30) avant la détermination de la première vitesse du son (c1) de la région intermédiaire (30), la première vitesse du son étant basée sur la pluralité de premières données d'image, chacune des premières données d'images étant déterminée sur la base d'un algorithme de formation de faisceau appliqué au moins aux signaux détectés correspondant à la première zone représentative (42) et qui prend comme paramètre une première vitesse de référence du son (c ref 1), et l'une d'une pluralité de premières vitesses supposées du son (c_supp_1).

22

**4.** Procédé selon l'une quelconque de la revendication 2 ou 3, comprenant en outre le calcul d'une pluralité de données d'images cibles associées à une zone cible représentative (44) de la région cible (12) avant la détermination de la vitesse cible du son (cT) à l'intérieur de la région cible (12), la vitesse cible du son (cT) étant basée sur la pluralité de données d'images cible dans la zone représentative (44) de la région cible (12), chacune des données d'image cibles étant déterminée sur la base d'un algorithme de formation de faisceau appliqué au moins aux signaux détectés correspondant à la zone représentative (44) et qui prend comme paramètres la position de l'interface (34), la vitesse cible de référence du son (c_ref_tar) appliquée à la région cible (12), la première vitesse du son (c1) appliquée à la région intermédiaire (30), et l'une de la pluralité de vitesses cibles supposées du son (c_supp_tar) pour la région cible (12).

**5.** Procédé selon l'une quelconque des revendications 1 à 4, dans lequel au moins l'un parmi :

- la pluralité de premières vitesses supposées du son (c_supp_1) et la pluralité de vitesses cibles supposées du son (c_supp_tar) ont une même valeur, et
- les premières vitesses supposées du son (c_supp_1) sont des vitesses connues du son pour la région intermédiaire (30), et les vitesses cibles supposées du son (c_supp_tar) sont des vitesses connues du son pour la région cible (12).

**6.** Procédé selon l'une quelconque des revendications 1 à 5, dans lequel la détermination de la position de l'interface (34) est basée sur au moins une partie des signaux détectés.

**7.** Procédé selon l'une quelconque des revendications 1 à 5, dans lequel la détermination de la position de l'interface (34) est basée sur un traitement d'image automatique de l'image morphologique.

**8.** Procédé selon l'une quelconque des revendications 1 à 6, dans lequel la position de l'interface (34) est déterminée sur la base de variations d'amplitudes de données d'image du milieu (14) le long de la direction de profondeur (D) entre la région intermédiaire (30) et la région cible (12), les données d'image étant déterminées sur la base des signaux détectés et d'un algorithme de formation de faisceaux qui prend comme paramètre la vitesse de référence du son (c0).

**9.** Procédé selon l'une quelconque des revendications 1 à 8, dans lequel la première vitesse du son (c1) et/ou la vitesse cible du son (cT) sont chacune calculées à l'aide d'un premier critère de focalisation et/ou d'un critère de focalisation cible respectifs, une pluralité de premières valeurs de focalisation et/ou de valeurs de focalisation cibles respectives étant obtenues par application du premier critère de focalisation et/ou du critère de focalisation cible respectifs à, respectivement, la pluralité de premières données d'image de la première zone représentative (42) et/ou la pluralité de données d'image cibles de la zone cible représentative (44), la première vitesse du son (c1) étant une sélectionnée de la pluralité de premières valeurs de focalisation respectives et/ou la vitesse cible du son (cT) étant une sélectionnée de la pluralité de valeurs de focalisation cibles respectives.

**10.** Procédé selon la revendication 9, dans lequel la première vitesse du son (c1) est le maximum de la pluralité de premières valeurs de focalisation respectives et/ou la vitesse cible du son (cT) est le maximum de la pluralité des valeurs de focalisation cibles respectives.

**11.** Procédé selon la revendication 9 ou 10, dans lequel le critère de focalisation est un critère de cohérence.

**12.** Procédé selon l'une quelconque des revendications 1 à 11, comprenant en outre :

- la détermination d'une position d'une sous-interface dans la région intermédiaire (30), la sous-interface divisant dans la direction de la profondeur la région intermédiaire (30) en une seconde région de la région intermédiaire proximale de la surface externe et une première région de la région intermédiaire proximale de l'interface (34),
- la détermination d'une seconde vitesse du son (c2) de la seconde région basée sur au moins une partie des signaux détectés en prenant comme paramètre une seconde vitesse de référence du son (c_ref_2), et l'une d'une pluralité de secondes vitesses supposées du son (c_supp_2),

dans lequel
la détermination de la première vitesse du son (c1) est basée sur au moins une partie des signaux détectés et prend en considération la position de la sous-interface, la première vitesse de référence du son (c_ref_1) appliquée à la première région, la seconde vitesse de référence du son (c_ref_2) appliquée à la seconde région, et une l'une de la pluralité de premières vitesses supposées du son (c_supp_1) pour la première région.

13. Procédé selon la revendication 12, dans lequel la détermination de la position de la sous-interface est basée sur au moins une partie des signaux détectés.

14. Procédé selon l'une quelconque des revendications 1 à 13, dans lequel la seconde région de la région intermédiaire contient la seconde zone représentative, et la première région de la région intermédiaire contient la première zone représentative.

15. Procédé selon l'une quelconque des revendications 1 à 14, comprenant en outre le calcul d'une pluralité de secondes données d'image associées à une seconde zone représentative de la seconde région avant la détermination de la seconde vitesse du son (c2) de la seconde région basée sur la pluralité de secondes données d'image, chacune des secondes données d'image étant déterminée sur la base d'un algorithme de formation de faisceau au moins appliqué aux signaux détectés correspondant à la seconde zone représentative et qui prend comme paramètre une seconde vitesse de référence du son (c_ref_2), et l'une d'une pluralité de secondes vitesses supposées du son (c_supp_2).

16. Procédé selon l'une quelconque des revendications 1 à 15, dans lequel le milieu (14) est un corps de mammifère et la surface externe (26) est la peau du mammifère, et
dans lequel la région cible (12) est le foie du mammifère, la région intermédiaire (30) est une région du milieu (14) compris entre le foie et la peau dans la direction de la profondeur (D).

17. Système d'imagerie par ultrasons (10) pour déterminer une vitesse cible du son (cT) à l'intérieur d'une région cible (12) d'un milieu (14), ledit système d'imagerie par ultrasons (10) comprenant :

- une sonde (20) apte à être placée fonctionnellement en contact avec une surface externe (26) du milieu (14), la sonde (20) étant apte à transmettre des ondes d'excitation (21) dans le milieu (14) dans une direction de la profondeur (D) vers la région cible (12), lesdites ondes d'excitation (21) étant rétrodiffractées dans le milieu (14) vers la sonde (20), la sonde (20) étant apte à détecter les ondes rétrodiffractées (29) et à fournir des signaux détectés correspondant au système ultrasonore (10), et
- une unité de traitement (16) mettant en œuvre le procédé selon l'une quelconque des revendications 1 à 16.

FIG. 1

FIG. 2

40

Determining position of an
interface between a first region
and a target region — S1

↓

Calculating a plurality of first image
data in the first region,
and determining a first speed of
sound with the first image data — S2

↓

Calculating a plurality of target image
data in the target region based
on the interface and the first speed
of sound, and determining a target
speed of sound in the target region — S3

# FIG. 3

FIG. 4

FIG. 5

D1

D

32  28  20  54

26

21  50

29  52

42

44

21

48

34

30

14

12

FIG. 6

**FIG. 7**

**EP 3 632 330 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130178740 A1
- US 2011082372 A1

- US 2018012545 A1

**Non-patent literature cited in the description**

- **MARION IMBAULT et al.** Robust sound speed estimation for ultrasound-based hepatic steatosis assessment. *Physics in Medecine & Biology,* 2017